Europäisches Patentamt

European Patent Office

Office européen des brevets

(11) Numéro de publication: **0 263 048 B1**

# FASCICULE DE BREVET EUROPEEN

(45) Date de publication de fascicule du brevet:
18.12.91

(51) Int. Cl.5: **C12N 7/00, C12N 7/08, A61K 39/12**

(21) Numéro de dépôt: 87460013.3

(22) Date de dépôt: 16.07.87

(54) Procédé de culture du virus de la rhinotrachéite infectieuse notamment de la dinde, et vaccin préparé à partir du virus ainsi obtenu.

(30) Priorité: 18.08.86 FR 8611872

(43) Date de publication de la demande:
06.04.88 Bulletin 88/14

(45) Mention de la délivrance du brevet:
18.12.91 Bulletin 91/51

(84) Etats contractants désignés:
AT BE CH DE ES FR GB GR IT LI LU NL SE

(56) Documents cités:
EP-A- 0 013 188

THE VETERINARY RECORD, vol. 119, no. 24, 13 décembre 1986, pages 599-600; R.C. JONES et al.: "Short Communications - Demonstration of a candidate virus for turkey rhinotracheitis in experimentally inoculated turkeys"

F. FENNER et al.: "Medical Virology", 1970, Academic Press, New York, US;

(73) Titulaire: Ministère de l'Agriculture Direction de la Qualité, Services Vétérinaires Labora-toire National de Pathologie Aviaire
Les Croix
F-22440 Ploufragan(FR)

Titulaire: RHÔNE MERIEUX
17, rue Bourgelat
F-69002 Lyon(FR)

(72) Inventeur: Giraud, Patrick
52 rue Louis Jouvet
F-22000 Saint-Brieuc(FR)
Inventeur: Bennejean, Georges
4 boulevard Lamartine
F-22000 Saint-Brieuc(FR)
Inventeur: Guittet, Michèle
11 bis rue Coetlogon
F-22000 Saint-Brieuc(FR)
Inventeur: Toquin, Didier
Bourg de Saint-Donan
F-22800 Quintin(FR)

(74) Mandataire: Dubreuil, Annie et al
Cabinet Ballot-Schmit 23, rue des Peupliers
B.P. 855
F-56108 Lorient Cédex(FR)

Rank Xerox (UK) Business Services

## Description

L'invention concerne un procédé de culture du virus de la rhinotrachéite infectieuse de la dinde qui est responsable également d'affections d'autres espèces aviaires telles que le syndrome infectieux des grosses têtes (ou de gonflement de la tête) de la poule et de la pintade. Ce virus sera appelé RTI/SIGT dans la suite de la description. L'invention concerne également tout vaccin préparé à partir du virus obtenu par ce procédé. Ces affections sont responsables de pertes économiques dues aux mauvaises performances, aux mortalités et aux saisies en abattoir. Ces maladies contagieuses diffusent rapidement sur tous les animaux d'un même bâtiment et d'un élevage à l'autre.

La mise au point d'une méthode de prévention de ces maladies s'avère donc tout à fait indispensable.

Le virus RTI/SIGT a été isolé par les demandeurs chez des dindonneaux atteints précisément de rhinotrachéite infectieuse, et chez des poulets et des pintades atteints de syndrome infectieux des grosses têtes. Le virus est notamment décrit par GIRAUD Patrick, BENNEJEAN Georges, GUITTET Michèle et TOQUIN Didier dans la "Semaine Vétérinaire" (1986, 413, 1-3).

Cet agent viral a tout d'abord été mis en évidence à partir de prélèvements sur des animaux malades ou sur des animaux autopsiés, par culture sur anneaux de trachée d'embryon de dinde exempts d'organismes pathogènes spécifiés (EOPS), ou de poulet (EOPS), chez lesquels le virus provoque un arrêt des battements ciliaires en 5 à 8 jours.

L'observation au microscope électronique de cet agent viral a été possible après ultracentrifugation du milieu de culture des anneaux de trachée: le virus se présente sous l'aspect de particules enveloppées et recouvertes de spicules. La morphologie de ce virus sera d'ailleurs décrite ci-dessous avec plus de précisions.

L'ensemble des caractéristiques de ce virus conduit à penser qu'il semble être un pseudo-myxovirus et, plus généralement, appartenir à la famille des Paramyxoviridae.

Les demandeurs ont observé qu'après l'infection par le virus RTI/SIGT et la guérison, les oiseaux deviennent résistants à une nouvelle infection, ce qui signifie que les oiseaux ont une réponse immunitaire d'excellente qualité à l'infection par le virus et peuvent donc être protégés de la maladie par vaccination. Or, pour réaliser un tel vaccin de manière optimale, on doit pouvoir cultiver ce virus dans des conditions de rendement compatibles avec les lois du marché. Certes, des procédés déjà connus de culture de virus ont été essayés: il s'agit par exemple de la culture sur anneaux de trachée d'embryon de poulet ou de dinde, ou de la culture sur des oeufs embryonnés de poule ou de dinde. Malheureusement, de telles cultures appliquées au virus RTI/SIGT se sont révélées trop peu efficaces pour que la fabrication d'un vaccin s'avère ensuite envisageable dans des conditions acceptables de rentabilité.

La présente invention a précisément pour objet de pallier ces inconvénients et concerne un nouveau procédé de culture du virus RTI/SIGT conduisant à une multiplication importante du virus. Elle concerne également tout vaccin obtenu à partir du virus RTI/SIGT ainsi cultivé.

La présente invention concerne plus précisément un procédé de culture du virus RTI/SIGT caractérisé en ce que le substrat de culture est constitué d'une lignée cellulaire continue de rein de singe.

La présente invention sera mieux comprise à l'aide des explications et des exemples qui vont suivre.

Selon une caractéristique importante de l'invention et conformément à une expérimentation faite par les demandeurs, la culture du virus RTI/SIGT est réalisée sur des lignées cellulaires continues de rein de singe, plus particulièrement sur des cellules de rein de singe African Green (Vero, BSC-1), Rhésus (LLC-MK2, MA 104) et Buffalo Green (BGM).

Le procédé selon l'invention consiste à ensemencer des cultures cellulaires de ces lignées continues de rein de singe, à procéder à l'incubation des cultures en présence du virus RTI/SIGT et à la récolte du virus après multiplication dans ces cellules.

Les principales étapes du procédé seront d'ailleurs décrites ci-dessous avec plus de précisions. Un tel procédé conforme à l'invention permet de produire une grande quantité de virus, utilisable comme antigène dans la préparation d'un vaccin contre les affections dues au virus RTI/SIGT dans toutes les espèces aviaires, et d'un réactif pour une technique sérologique de mise en évidence des anticorps dirigés contre le virus.

Selon une caractéristique importante de l'invention, les souches virales obtenues par le procédé décrit précédemment de culture du virus RTI/SIGT sur cellules de rein de singe, sont utilisées pour la fabrication de vaccins selon les méthodes décrites ci-dessous à titre d'exemple nullement limitatif.

Pour produire un vaccin, on utilise le virus privé de son pouvoir pathogène, sans qu'il ait perdu son activité antigénique, c'est-à-dire sa capacité à stimuler la production d'anticorps, ou encore à entraîner une réponse immunitaire. Pour cela, on peut atténuer le virus ou l'inactiver.

Pour préparer un vaccin à virus vivant atténué contre les affections dues au virus RTI/SIGT définies précédemment, on effectue avec le virus un nombre minimum de passages nécessaires à cette atténuation, par exemple supérieur à 10 et pouvant aller jusqu'à 200, sur cellules de rein de singe, selon le procédé décrit précédemment. Après avoir vérifié l'atténuation du pouvoir pathogène du virus par inoculation à l'animal sensible, dindonneau (EOPS), poulet (EOPS) par exemple, on peut produire le vaccin sur cellules de rein de singe selon le procédé conforme à l'invention décrit précédemment.

Pour préparer un vaccin à virus inactivé contre les affections dues au virus RTI/SIGT dans toutes les espèces aviaires, il est nécessaire de cultiver le virus comme décrit précédemment sur cellules de rein de singe, afin d'obtenir un titre élevé. On peut d'ailleurs sélectionner un clone viral approprié aux exigences de fabrication. On effectue ensuite une inactivation du virus selon les procédés classiques d'inactivation de virus, décrits ci-dessous.

Une caractéristique importante du vaccin selon l'invention est de pouvoir être indifféremment utilisé pour l'immunisation de toutes espèces aviaires, ceci indépendamment de l'espèce aviaire d'origine du virus utilisé pour la préparation du vaccin

Avant de développer les applications du procédé de culture et de multiplication du virus RTI/SIGT décrit précédemment, il convient de caractériser ce virus:

1. Le virus possède un acide nucléique de type ARN; la multiplication du virus dans les cellules de rein de singe n'est pas influencée de façon significative par l'addition d'IUdR (5-iodo-uracile- 2'-désoxyriboside) dans le milieu de culture.

2. Le virus est sensible à l'action des solvants des lipides; le traitement du surnageant des cultures de cellules de rein de singe infectées par le virus avec de l'éther ou du chloroforme entraîne une diminution significative du titre infectieux. Ceci est une caractéristique des virus enveloppés qui contiennent des lipides.

3. L'examen au microscope électronique de la préparation virale provenant du surnageant des cultures de cellules de rein de singe infectées par le virus permet de mettre en évidence des particules enveloppées très polymorphes et recouvertes de spicules. Le virus est constitué par une nucléocapside centrale disposée en pelote. Le virus possède des dimensions très variables; le diamètre moyen varie de 100 à 200 nanomètres avec des extrêmes variant de 70 à 600 nanomètres environ. On obse rve des formes circulaires, ovales, filamenteuses ou très irrégulières. Les spicules ont une longueur d'environ 12 à 15 nanomètres. La nucléocapside apparaît comme une structure hélicoïdale de 15 nanomètres environ de diamètre.

Les cellules de rein de singe infectées par le virus en coupes ultra-fines examinées au microscope électronique permettent d'observer le bourgeonnement des virions au niveau de la membrane cellulaire externe localement épaissie par l'accumulation du matériel viral. On observe également l'accumulation de matériel viral dans les inclusions intracytoplasmiques.

4. Les tests sérologiques pour la recherche des anticorps dirigés contre le virus RTI/SIGT sont nombreux. On peut citer: la séroneutralisation sur culture cellulaire de rein de singe, ou autre substrat de culture, l'immunofluorescence sur cultures cellulaires de rein de singe infectées, et la méthode sérologique connue sous le nom d'E.L.I.S.A. avec un antigène fabriqué sur cellules de rein de singe. Ces différentes techniques ne révèlent aucune communauté antigènique entre le virus RTI/SIGT et les autres virus aviaires ou des virus de mammifères proches morphologiquement.

5. Le virus ne possède pas la propriété spontanée d'hémagglutiner (hématies de poulet, dinde, cheval, bovin, canard, mouton, cobaye, porc et homme). Le virus ne possède pas non plus d'activité neuraminidasique décelable.

6. L'inoculation expérimentale par voie intranasale du virus pathogène non atténué sur cellules de rein de singe permet de reproduire une rhi- notrachéite infectieuse caractéristique sur din- donneaux (EOPS), et permet de reproduire les symptômes préliminaires du SIGT chez le poulet et le pintadeau. Des lésions histologiques caractéristiques correspondant à des inclusions intracytoplasmiques éosinophiles sont observées dans les cellules ciliées de l'épithélium des voies respiratoires supérieures (trachée, larynx, cavités nasales).

Les caractéristiques de ce virus inclinent donc à penser, comme cela a été dit précédemment, qu'il appartient bien au groupe des pseudomyxovirus et plus généralement, à la famille des Paramyxoviridae.

Le procédé décrit précédemment concernant la culture, la multiplication, l'atténuation du virus RTI/SIGT ainsi caractérisé, conduisant notamment à l'obtention du vaccin, est maintenant illustré au moyen d'exemples non limitatifs donnés ci-après.

**EXEMPLE I :** Isolement, culture et multiplication du virus RTI/SIGT sur cellules de rein de singe.

Des échantillons sont prélevés sur des animaux malades (écouvillons de trachée, aspiration de mucosités nasales...) ou sur des animaux autopsiés (broyats d'organes de l'appareil respiratoire, surtout des voies supérieures...). Les échantillons, conservés à basse température (par exemple

+ 4° C), sont suspendus dans un milieu de transport contenant des antibiotiques (on peut utiliser comme milieu, de l'eau peptonée, du tampon "phosphate"...). On filtre les suspensions.

Les filtrats sont ensuite inoculés à des lignées cellulaires continues de rein de singe. On utilise plus particulièrement des cellules communément appelées "VERO" provenant de rein de singe African Green, du type de celles qui sont commercialisées par les laboratoires FLOW sous le numéro ATCC: CCL.81, et entretenues au-delà du 130ème passage.

Pour une boîte de 25 cm², on prépare 10 ml d'une suspension cellulaire à la concentration de 250.000 cellules VERO par millilitre. On ensemence 100 microlitres de prélèvement par boîte et de préférence sur tapis cellulaire non encore établi. Le milieu utilisé peut être du milieu essentiel minimum de Eagle avec sels de Earle, additionné de tampon Hépès, de sérum de veau foetal et d'antibiotiques. On incube à 37° C. On observe au bout de plusieurs jours la présence du virus par l'apparition d'un effet cytopathique.

Selon une première variante, on effectue au bout de quelques jours une subculture du tapis infecté. Selon une seconde variante, on effectue une co-culture de cellules infectées et de cellules saines. Dans les deux cas, on obtient un effet cytopathique plus important en quelques jours.

L'effet cytopathique (lésions du tapis cellulaire) se traduit par des cellules ballonnées, arrondies, réfringentes, qui se décollent et flottent dans le surnageant de culture. Après diverses méthodes de coloration (May-Gründwald-Giemsa, etc.), on note l'apparition d'une volumineuse ou de plusieurs inclusions intracytoplasmiques qui repoussent légèrement le noyau. Par coloration à l'hemalun-éosine, l'inculsion virale apparaît éosinophile.

Le noyau ne présente pas d'inclusion, mais parfois une margination de la chromatine, bien visible en coloration à l'acridine orange. Le virus entraîne également l'apparation de syncytia.

Ce virus est mis en évidence dans le surnageant de culture par microscopie électronique. Ce virus est ensuite récolté et stocké à - 70° C.

L'isolement ayant été réalisé comme décrit précédemment, la souche virale peut être ensuite adaptée par quelques passages successifs sur le substrat choisi, par exemple des cellules VERO. Une fois bien adaptée, la souche entraîne un effet cytopathique bien visible en quelques jours. Ce procédé de culture sur cellules de rein de singe, notamment sur cellules VERO, assure une multiplication satisfaisante du virus RTI/SIGT.

**EXEMPLE II :** Préparation d'un vaccin vivant atténué à partir du virus RTI/SIGT.

Pour la préparation d'un vaccin vivant atténué, il est nécessaire de procéder à l'atténuation du virus RTI/SIGT. L'isolement et la multiplication étant effectués selon l'exemple I, on effectue pour atténuer la souche virale un très grand nombre de passages sur le substrat de culture constitué conformément à l'invention par des cellules de rein de singe. Ce nombre de passages peut varier de 10 à 200 par exemple. L'atténuation du pouvoir pathogène du virus peut être vérifiée par inoculation à l'animal sensible, dindonneau (EOPS) et poulet (EOPS) par exemple.

Après avoir multiplié la souche virale atténuée sur cellules de rein de singe, on recueille les surnageants de culture et/ou les cellules. On peut soumettre les cellules récoltées à un traitement ultrasonique. On conserve la suspension virale à basse température (au minimum -20° C, mais de préférence - 70° C). On peut ajouter un agent stabilisant tel que les hydrates de carbone (sorbitol, mannitol, amidon, saccharose, dextrane, glucose...), les protéines (albumines, caséine...), un agent contenant des protéines (sérum bovin, lait écrémé...) et un tampon (phosphate de métal alcalin...). Comme agent stabilisant, on peut utiliser le mélange décrit par Bovarnick sous le nom de SPGA (Journal. Bact. 1950, 59, 509).

On peut lyophiliser la préparation après avoir ajouté un stabilisateur et la conserver sous vide ou sous azote; on peut ajouter un adjuvant tel que l'hydroxyde d'aluminium.

**EXEMPLE III :** Préparation d'un vaccin inactivé à partir du virus RTI/SIGT.

Si le virus est destiné à la préparation d'un vaccin inactivé, on procède de la manière décrite ci-après. L'isolement et la multiplication ayant été effectués suivant l'exemple I, on titre la préparation virale. On effectue l'inactivation par du formaldéhyde (à la concentration finale de 0,2 à 0,4 % pendant 24 heures à 20° C ou 2 heures à 37° C), par des solvants organiques (hydrocarbures halogénés en présence d'un tensio-actif tel qu'un mono-oléate de polyoxyéthylène-sorbitane), par la béta-propiolactone, par l'éthylène-imine ou par leurs dérivés. On peut également inactiver le virus par clivage selon divers procédés, par exemple l'utilisation d'une enzyme et/ou d'un solvant organique.

Après l'inactivation, on peut neutraliser l'agent d'inactivation (par exemple le formaldéhyde par du thiosulfate) puis on ajuste le pH à 7. Un échantillon est testé afin de contrôler l'inactivation, en recherchant notamment l'absence d'effet cytopathique sur cultures cellulaires de rein de singe. On stocke à basse température (par exemple - 20° C). Enfin, on mélange le virus inactivé à un adjuvant. L'adjuvant peut être par exemple l'hydroxyde d'aluminium ou une composition constituée d'une huile minérale et d'un ou plusieurs émulsifiants.

La suspension virale est émulsifiée dans la phase huileuse de l'adjuvant.

**EXEMPLE IV :** Administration des vaccins préparés à partir du virus RTI/SIGT.

On administre les vaccins aux espèces aviaires sensibles à la maladie. On peut vacciner de l'âge d'un jour jusqu'à l'entrée en période de ponte et effectuer des rappels de vaccination. L'administration de vaccin atténué se fait par nébulisation, par addition à l'eau de boisson, par dépôt d'une gouttelette dans l'oeil ou les narines, par écouvillonage trachéal ou cloacal, ou encore par injection. Le vaccin inactivé doit être administré par injection, par exemple par voie sous-cutanée ou intramusculaire.

Par les procédés décrits précédemment concernant la culture, la multiplication, l'atténuation du virus RTI/SIGT, la préparation d'antigène pour la vaccination et la sérologie, l'invention s'applique à la détection et à la prévention de la rhinotrachéite infectieuse de la dinde, du syndrome infectieux des grosses têtes de la poule et de la pintade et des affections dues au virus RTI/SIGT dans les autres espèces aviaires telles que le corysa de la pintade et les maladies respiratoires du canard, de l'oie, du faisan, de la caille, etc.

Il convient de noter qu'une souche du virus de la rhinotrachëite infectieuse de la dinde, décrit précédemment, a été déposée à la Collection Nationale de Cultures de Microorganismes de l'Institut Pasteur, 25 Rue du Docteur Roux, 75724 PARIS Cedex 15 sous les références suivantes:
Souche Virale 86004
Lot (OVS 5)
déposée le 9 octobre 1986
sous le numéro I 611

**Revendications**

1. Procédé de culture du virus de la rhinotrachéite infectiose de la dinde (RTI/SIGT), caractérisé en ce que le substrat de culture est constitué d'une lignée cellulaire continue de rein de singe.

2. Procédé de culture selon la revendication 1, caractérisé en ce que ce substrat est utilisé pour la multiplication de ce virus.

3. Procédé de culture selon l'une des revendications 1 et 2, caractérisé en ce que ce substrat est utilisé pour l'atténuation de ce virus.

4. Vaccin destiné à l'immunisation d'espèces aviaires contre le virus RTI/SIGT, caractérisé en ce qu'il est préparé à partir de souches virales obtenues par le procédé selon l'une des revendications 1, 2 et 3.

5. Vaccin selon la revendication 4, caractérisé en ce que ces souches sont atténuées par un grand nombre de passages sur ce substrat, le vaccin étant dans ce cas un vaccin à virus vivant.

6. Vaccin selon la revendication 4, caractérisé en ce que ces souches sont inactivées, le vaccin étant dans ce cas un vaccin à virus inactivé.

7. Vaccin selon l'une des revendications 4, 5 et 6, caractérisé en ce qu'il est destiné indifféremment à l'immunisation de toutes espèces aviaires, ceci indépendamment de l'espèce aviaire d'origine du virus utilisé pour la préparation du vaccin.

**Claims**

1. A method of culturing infectious turkey rhinotracheitis virus (RTI/SIGT), characterized in that the culture substrate is constituted by a continuous strain of monkey renal cells.

2. The method as claimed in claim 1, characterized in that the substrate is employed for the multiplication of the said virus.

3. The method as claimed in claim 1 or in claim 2, characterized in that the substrate is used for the attenuation of the said virus.

4. A vaccine intended for the immunization of avian species against the virus RTI/SIGT, characterized in that it is prepared from virus strains obtained by the method as claimed in any one of the preceding claims 1 through 3.

5. The vaccine as claimed in claim 4, characterized in that the said strains are attenuated by a large number of passes through the said substrate, the vaccine being in this case a living virus vaccine.

6. The vaccine as claimed in claim 4, characterized in that the said strains are inactivated, the vaccine then in this case being an inactivated virus vaccine.

7. The vaccine as claimed in any one of the preceding claims 4, 5 and 6, characterized in that it is intended in like manner for the immunization of all avian species independently of the avian species which is the origin of the virus used for the preparation of the vaccine.

**Patentansprüche**

1. Verfahren zum Kultivieren des Virus der infektiösen Rhinotracheitis der Truthenne (RTI/SIGT), **dadurch gekennzeichnet,** daß das Kultursubstrat aus einem kontinuierlichen Affennieren-Zellstamm gebildet wird.

2. Kulturverfahren nach Anspruch 1, **dadurch gekennzeichnet,** daß das Subtrat zur Vermehrung des Virus verwendet wird.

3. Kulturverfahren nach einem der Ansprüche 1 oder 2, **dadurch gekennzeichnet,** daß das Substrat zur Abschwächung des Virus verwendet wird.

4. Impfstoff zur Immunisierung von Vogelarten gegen das RTI/ SIGT-Virus, **dadurch gekennzeichnet,** daß er ausgehend von Virusstämmen, die durch das Verfahren gemäß einem der Ansprüche 1 bis 3 erhalten wurden, hergestellt worden ist.

5. Impfstoff nach Anspruch 4, **dadurch gekennzeichnet,** daß die Stämme durch eine große Zahl von Passagen über das Substrat abgeschwächt worden sind, wobei der Impfstoff in diesem Falle ein Lebendvirusimpfstoff ist.

6. Impfstoff nach Anspruch 4, **dadurch gekennzeichnet,** daß die Stämme inaktiviert worden sind, wobei der Impfstoff in diesem Falle ein Impfstoff mit inaktivierten Viren ist.

7. Impfstoff nach einem der Ansprüche 4 bis 6, **dadurch gekennzeichnet,** daß er gleichermaßen zur Immunisierung sämtlicher Vogelarten bestimmt ist, unabhängig von der Vogelart der Herkunft des Virus, das zur Herstellung des Impfstoffs verwendet worden ist.